# EUROPEAN PATENT APPLICATION

(11) **EP 4 140 488 A1**
(43) Date of publication of application: **01.03.2023**
(21) Application number: 21791632.9
(22) Date of filing: 06.04.2021
(51) Int. Cl.: A61K 31/5415, A61K 31/713, A61P 13/12, A23L 33/10

(54) **COMPOSITION FOR PREVENTING OR TREATING ISCHEMIA REPERFUSION INJURY COMPRISING NADPH OXIDASE 1 INHIBITOR AS ACTIVE INGREDIENT**

(30) Priority: 20.04.2020 KR 20200047663
(71) Applicant: Kyungpook National University Industry-Academic Cooperation Foundation, Daegu 41566 (KR); Kyungpook National University Hospital, Daegu 41944 (KR)
(72) Inventor: CHO, Jang-Hee, Daegu 41944 (KR); JUNG, Hee-Yeon, Daegu 41944 (KR); OH, Se-Hyun, Daegu 41944 (KR)
(74) Representative: Schwarz & Partner Patentanwälte GmbH
(86) International application number: PCT/KR2021/004245
(87) International publication number: WO 2021/215700

(57) **Abstract**

The present invention relates to a composition for preventing or treating ischemia -reperfusion injury, the composition comprising an NADPH oxidase 1 (NOX1) inhibitor as an active ingredient, and more specifically, to a composition exhibiting prophylactic or therapeutic effects on ischemia reperfusion injury by suppressing the signaling of extracellular-signal-regulated kinase (ERK) by means of reactive oxygen species (ROS).

## Description

### THECHNICAL FIELD

This application claims priority to Korean Patent Application No. 10-2020-0047663 filed on April 20, 2020, and the entire specifications of which are incorporated herein by reference in their entireties.

The present invention relates to a composition for preventing or treating ischemia-reperfusion injury comprising a NADPH oxidase 1 (NOX1) inhibitor as an active ingredient, more particularly, it relates to a composition characterized in that it exhibits an effect of preventing or treating ischemia-reperfusion injury through inhibition of extracellular-signal-regulated kinase (ERK) signal transduction by reactive oxygen species (ROS).

### BACKGROUND OF THE INVENTION

Blood supply to specific tissues may be restricted during surgical procedures such as organ transplant surgery or surgery for the treatment of cardiovascular diseases, in this case, damage due to ischemia occurs as blood circulation is blocked in organs that require continuous blood flow, such as the liver, kidney, heart, and brain. In addition, if blood flow is suddenly increased due to reperfusion in an ischemia state in which oxygen is not supplied properly, cells and tissues may be greatly damaged due to various complex causes. In particular, liver or renal ischemia-reperfusion injury (IRI) is a major complication that occurs frequently after liver or kidney transplantation or cardiac surgery. During surgery, blood supply to the liver or kidneys is temporarily stopped due to blockage of blood flow, and subsequent reperfusion may induce a severe acute inflammatory response and acute tissue damage. In particular, such acute inflammatory reaction or cell apoptosis caused by damage to breakfast is one of the main causes of liver failure or renal failure, so it is recognized as a very serious risk factor. Ischemic reperfusion injury is also caused by a sudden increase in intracellular calcium concentration due to an abrupt increase in blood flow during oxygen deprivation. Increased intracellular calcium can mediate mitochondrial damage, at this time, substances released by mitochondrial damage react with ATP to generate reactive oxygen species, which the human body recognizes as inflammation and attacks white blood cells, generating more reactive oxygen species, which can eventually lead to cell damage. Ischemic reperfusion injury is more severe as the speed of blood reflow increases, and ischemic reperfusion injury frequently occurs when blood flow is resumed after cardiac surgery or organ transplantation. As such, when ischemic reperfusion injury is predicted, research results have been reported that it can be prevented by pre-administering a powerful antioxidant as a treatment for the damage caused by free radicals as one of the main factors. However, strong antioxidants have also been used limitedly due to limitations in effectiveness, and clinical studies on some drugs are in progress, but drugs that directly prevent or treat ischemic reperfusion injury have not been developed so far. Surgical procedures that can cause ischemic reperfusion tissue damage, such as organ transplantation, are rapidly increasing worldwide, and there is an urgent need medically and socially to develop a new technique that can restore organ function while suppressing inflammatory response and cell death after ischemia-reperfusion injury, which is a problem during liver or kidney transplantation.

On the other hand, ischemia reperfusion injury that occurs during kidney transplant surgery is associated with progressive loss of function and dysfunction of the transplanted kidney later. In addition, it has been clinically found that early ischemic reperfusion injury and acute rejection are correlated with the long-term prognosis at transplantation. Although the exact mechanism causing ischemia reperfusion injury has not been elucidated, it is known that the generation of oxygen free radicals by ischemia reperfusion is one of the important factors. In addition, it is reported that ischemia/reperfusion injury during kidney transplantation causes functional delay during transplantation and is also associated with acute and chronic rejection. In acute renal failure caused by ischemic injury, it is known that, in addition to factors such as vasoconstriction and tubular occlusion, the mechanisms of inflammatory and cytotoxic damage accompanied by increased inflammatory cytokines and neutrophil infiltration into the renal parenchyma are known to play an important role.

Renal ischemic reperfusion injury (IRI) in kidney transplant surgery is a cause of increased fatal patient complications and mortality after surgery. To prevent this, methods such as ischemic preconditioning, pretreatment with adenosine, preconditioning of inhaled mouth anesthetic, and AMP-activated protein kinase (AMPK) activation are being studied, it has been known so far that not only acute ischemic preconditioning but also delayed ischemic preconditioning protects against ischemic reperfusion injury of the kidney through intermediate mediators such as intracellular signaling proteins Akt and ERK, HSP27 (heat shock protein 27), HSP70, iNOS (inducible nitric oxide synthase). In addition, research was conducted to develop a drug that can treat ischemia-reperfusion injury of the kidney that occurs during a kidney transplant operation, but there were various side effects, and it was difficult to solve problems such as deterioration of kidney function caused by transplantation.

### DETAILED DESCRIPTION OF THE INVENTION

### TECHNICAL PROBLEM

Accordingly, the present inventors have made diligent efforts to develop a safe new material capable of alleviating tissue damage caused by ischemia-reperfusion, the present invention was completed by confirming that the NOX1 inhibitor exhibits a very excellent preventive or therapeutic effect on tissue damage caused by ischemia-reperfusion through inhibition of extracellular-signal-regulated kinase (ERK) signal transduction by reactive oxygen species (ROS).

Accordingly, an object of the present invention is to provide a pharmaceutical composition for preventing or treating ischemia-reperfusion injury, comprising an NADPH oxidase 1 (NOX1) inhibitor as an active ingredient.

Accordingly, an object of the present invention is to provide a pharmaceutical composition for preventing or treating ischemia-reperfusion injury, consisting of an NADPH oxidase 1 (NOX1) inhibitor as an active ingredient.

Accordingly, an object of the present invention is to provide a pharmaceutical composition for preventing or treating ischemia-reperfusion injury, essentially consisting of an NADPH oxidase 1 (NOX1) inhibitor as an active ingredient.

Another object of the present invention is to provide a food composition for preventing or improving ischemia-reperfusion injury comprising an NADPH oxidase 1 (NOX1) inhibitor as an active ingredient.

Another object of the present invention is to provide a food composition for preventing or improving ischemia-reperfusion injury consisting of an NADPH oxidase 1 (NOX1) inhibitor as an active ingredient.

Another object of the present invention is to provide a food composition for preventing or improving ischemia-reperfusion injury essentially consisting of an NADPH oxidase 1 (NOX1) inhibitor as an active ingredient.

Another object of the present invention is to provide a use of a NADPH oxidase 1 (NOX1) inhibitor for the manufacture of a preparation for the treatment of ischemia-reperfusion injury.

Another object of the present invention is to provide a method for treating ischemia-reperfusion injury comprising administering to a subject in need thereof an effective amount of a composition comprising an NADPH oxidase 1 (NOX1) inhibitor as an active ingredient.

### TECHNICAL SOLUTION

In order to achieve the above object of the present invention, the present invention provides a pharmaceutical composition for preventing or treating ischemia-reperfusion injury, comprising an NADPH oxidase 1 (NOX1) inhibitor as an active ingredient.

In addition, the present invention provides a pharmaceutical composition for preventing or treating ischemia-reperfusion injury consisting of a NADPH oxidase 1 (NOX1) inhibitor.

In addition, the present invention provides a pharmaceutical composition for preventing or treating ischemia-reperfusion injury essentially consisting of an NADPH oxidase 1 (NOX1) inhibitor.

In order to achieve another object of the present invention, the present invention provides a food composition for preventing or improving ischemia-reperfusion injury comprising an NADPH oxidase 1 (NOX1) inhibitor as an active ingredient.

In addition, the present invention provides a food composition for preventing or improving ischemia-reperfusion injury consisting of an NADPH oxidase 1 (NOX1) inhibitor.

In addition, the present invention provides a food composition for preventing or improving ischemia-reperfusion injury essentially consisting of an NADPH oxidase 1 (NOX1) inhibitor.

In order to achieve another object of the present invention, the present invention provides a use of an NADPH oxidase 1 (NOX1) inhibitor for manufacture of a preparation for the treatment of ischemia-reperfusion injury.

In order to achieve another object of the present invention, the present invention provides a method for treating ischemia-reperfusion injury comprising administering to a subject in need thereof an effective amount of a composition comprising an NADPH oxidase 1 (NOX1) inhibitor as an active ingredient.

Hereinafter, the present invention will be described in detail.

The present invention provides a pharmaceutical composition for preventing or treating ischemia-reperfusion injury comprising an NADPH oxidase 1 (NOX1) inhibitor as an active ingredient.

In the present invention, the type of the NOX1 inhibitor is not particularly limited, and any substance that exhibits a NOX1-dependent reactive oxygen species (ROX) generation inhibitory effect may be included in the inhibitor of the present invention. For example, the NOX1 inhibitor may be at least one selected from the group consisting of a single compound, a mixture of compounds (e.g., natural extracts, or cell or tissue cultures), and an antibody, peptides, proteins; or antisense nucleotides, siRNA, shRNA and ribozyme for NOX1 gene.

In one embodiment of the present invention, the NOX1 inhibitor may be a compound of Formula 1 below or a pharmaceutically acceptable salt thereof:

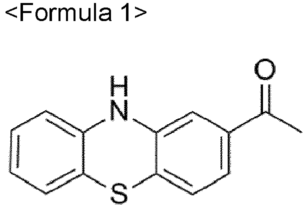

According to an embodiment of the present invention, it was confirmed that the compound of Formula 1 exhibits an effect of alleviating tissue damage due to ischemia-reperfusion by inhibiting reactive oxygen species-mediated ERK signal transduction in a mouse model induced by renal ischemia reperfusion.

As the salt in the present invention, an acid addition salt formed by a pharmaceutically acceptable free acid is useful. Acid addition salts are obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono and dicarboxylates, phenyl-substituted alkanoates, hydroxy alkanoates and alkanedioates, aromatic acids, aliphatic and aromatic sulfonic acids. Such pharmaceutically non-toxic salts include sulfates, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne -1,4-dioate, hexane-1,6-dioate, benzoate, chlorobenzoate, methylbenzoate, dinitro benzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, maleate, tartrate, methanesulfonate, propane sulfonate, naphthalene-1-sulfonate, naphthalene-2-sulfonate or mandelate.

The acid addition salt according to the present invention can be prepared by a conventional method, for example, by dissolving the compound represented by Formula 1 in an excess of an aqueous acid solution, and precipitating the salt using a water-miscible organic solvent such as methanol, ethanol, acetone or acetonitrile. It can also be prepared by evaporating the solvent or excess acid from the mixture to dryness, or by suction filtration of the precipitated salt.

In addition, a pharmaceutically acceptable metal salt can be prepared using a base. The alkali metal or alkaline earth metal salt is obtained, for example, by dissolving the compound in an excess alkali metal hydroxide or alkaline earth metal hydroxide solution, filtering the undissolved compound salt, and evaporating and drying the filtrate. In this case, it is pharmaceutically suitable to prepare a sodium, potassium or calcium salt as the metal salt. The corresponding silver salt is also obtained by reacting an alkali metal or alkaline earth metal salt with a suitable negative salt (e.g., silver nitrate).

The pharmaceutical composition according to the present invention may contain the NOX1 inhibitor alone or may be formulated in a suitable form together with a pharmaceutically acceptable carrier, and may further contain an excipient or diluent. As used herein, 'pharmaceutically acceptable' refers to a non-toxic composition that is physiologically acceptable and does not normally cause allergic reactions such as gastrointestinal disorders, dizziness, or similar reactions when administered to humans.

The pharmaceutically acceptable carrier may further include, for example, a carrier for oral administration or a carrier for parenteral administration. Carriers for oral administration may include lactose, starch, cellulose derivatives, magnesium stearate, stearic acid, and the like. In addition, various drug delivery materials used for oral administration of the peptide formulation may be included. In addition, the carrier for parenteral administration may include water, a suitable oil, saline, aqueous glucose and glycol, and the like, and may further include a stabilizing agent and a preservative. Suitable stabilizers include antioxidants such as sodium hydrogen sulfite, sodium sulfite or ascorbic acid. Suitable preservatives include benzalkonium chloride, methyl- or propyl-paraben and chlorobutanol. The pharmaceutical composition of the present invention may further include a lubricant, a wetting agent, a sweetening agent, a flavoring agent, an emulsifying agent, a suspending agent, and the like, in addition to the above components. For other pharmaceutically acceptable carriers and agents, reference may be made to those described in the art.

The composition of the present invention may be administered to mammals including humans by any method. For example, it can be administered orally or parenterally. The parenteral administration method is not limited thereto, but may be intravenous, Intramuscular, intraarterial, intramedullary, intrathecal, intracardiac, transdermal, subcutaneous, intraperitoneal, intranasal, enteral, topical, sublingual or rectal administration.

The pharmaceutical composition of the present invention may be formulated as a formulation for oral administration or parenteral administration according to the administration route as described above.

In the case of an agent for oral administration, the composition of the present invention may be formulated as a powder, granules, tablets, pills, dragees, capsules, liquids, gels, syrups, slurries, suspensions or the like, using a method known in the art. For example, oral preparations can be obtained by mixing the active ingredient with solid excipients, grinding them, adding suitable adjuvants, and processing them into a mixture of granules to obtain tablets or dragees. Examples of suitable excipients may include sugars including lactose, dextrose, sucrose, sorbitol, mannitol, xylitol, erythritol and maltitol and the like, starches including corn starch, wheat starch, rice starch and potato starch and the like, celluloses including cellulose, methyl cellulose, sodium carboxymethylcellulose and hydroxypropylmethyl-cellulose and the like, and fillers such as gelatin, polyvinylpyrrolidone, and the like. In addition, cross-linked polyvinylpyrrolidone, agar, alginic acid or sodium alginate may be added as a disintegrant if necessary. Furthermore, the pharmaceutical composition of the present invention may further include an anti-aggregating agent, a lubricant, a wetting agent, a flavoring agent, an emulsifying agent, and an antiseptic agent.

Agents for parenteral administration may be formulated in the form of injections or creams, lotion, external ointment, oil, moisturizer, gel, aerosol and nasal inhalant by methods known in the art. These formulations are described in formulary commonly known in all pharmaceutical chemistry.

The total effective amount of the pharmaceutical composition of the present invention may be administered to a patient as a single dose, and may be administered by a fractionated treatment protocol in which multiple doses are administered for a long period of time. The pharmaceutical composition of the present invention may vary the content of the active ingredient depending on the severity of the disease. Preferably, the total dose of the pharmaceutical composition of the present invention may be about 0.01 ug to 10,000 mg, most preferably 0.1 ug to 500 mg per 1 kg of the patient's body weight per day. However, since the dosage of the pharmaceutical composition of the present invention is determined in consideration of various factors such as the age, body weight, health status, sex, disease severity, diet and excretion rate etc. of the patient as well as the route of administration and the number of treatments, and the effective dosage for the patient is determined, considering this point, those of ordinary skill in the art will be able to determine an appropriate effective dosage according to the specific use of the pharmaceutical composition of the present invention as a therapeutic agent for neurodegenerative diseases. The pharmaceutical composition according to the present invention is not particularly limited in its formulation, administration route and administration method as long as the effect of the present invention is exhibited.

In the present invention, the type of organ exhibiting the ischemia-reperfusion injury is not particularly limited, but may be, for example, liver, heart, kidney, lung, brain, and most preferably, kidney.

The present invention also provides a food composition for preventing or improving ischemia-reperfusion injury, comprising an NADPH oxidase 1 (NOX1) inhibitor as an active ingredient.

The food composition of the present invention includes all types of functional food, nutritional supplement, health food, and food additives. Food compositions of this type can be prepared in various forms according to conventional methods known in the art.

For example, as a health food, the NOX1 inhibitor may be prepared in the form of tea, juice, and drink to drink, or may be ingested by granulation, encapsulation, and powder. In addition, it can be prepared in the form of a composition by mixing with a known substance or active ingredient known to have a preventive or therapeutic effect on ischemia-reperfusion tissue damage. For example, the food composition of the present invention may further contain a trace amount of minerals, vitamins, lipids, saccharides, and known components in addition to the NOX1 inhibitor component. The minerals may contain nutrients necessary for growth, such as calcium and iron, and vitamins may include vitamin C, vitamin E, vitamin B1, vitamin B2, vitamin B6, and the like. The lipid may include alkoxyglycerol or lecithin, and the saccharide may include fructooligosaccharide.

In addition, as a functional food, it can be prepared by adding the NOX1 inhibitor to beverages (including alcoholic beverages), fruits and their processed foods (e.g., canned fruit, canned food, jam, marmalade, etc.), fish, meat and their processed foods (e.g., ham, sausage corn beef, etc.), breads and noodles (e.g., udon, soba, ramen, spaghetti, macaroni, etc.), fruit juice, various drinks, cookies, syrup, dairy products (e.g., butter, cheese, etc.), edible vegetable oil, margarine, vegetable protein, retort food, frozen food, various seasonings (e.g., miso, soy sauce, sauce, etc.) and the like.

In addition, in order to use the NOX1 inhibitor of the present invention in the form of a food additive, it may be prepared and used in the form of a powder or a concentrate.

A preferred content of the NOX1 inhibitor in the food composition of the present invention may be 0.01 to 90%, preferably 0.1 to 50% based on the total weight of the food with respect to the total weight of the food composition.

The present invention provides a use of a NADPH oxidase 1 (NOX1) inhibitor for the manufacture of a preparation for the treatment of ischemia-reperfusion injury.

The present invention provides a method for treating ischemia-reperfusion injury comprising administering to a subject in need thereof an effective amount of a composition comprising an NADPH oxidase 1 (NOX1) inhibitor as an active ingredient.

The 'effective amount' of the present invention refers to an amount that, when administered to a subject, has an effect of improving, treating, preventing, detecting, diagnosing, or inhibiting or reducing an ischemia-reperfusion injury, and the 'subject' may be an animal, preferably an animal, including a mammal, particularly a human, and may be an animal-derived cell, tissue, organ, or the like. The subject may be a patient in need of the effect.

The 'treatment' of the present invention refers to improving ischemia-reperfusion injury or symptoms of the diseases comprehensively, and this may include curing, substantially preventing, or ameliorating the condition of the diseases, and include alleviating, curing or preventing one or most of the symptoms resulting from the disease, but it is not limited thereto.

As used herein, the term "comprising" is used synonymously with "including" or "characterized by", in the composition or method according to the present invention, additional components or steps of the method not specifically mentioned are not excluded. In addition, the term "consisting of" means excluding additional elements, steps, or ingredients not specifically described. The term "essentially consisting of" means that, in the scope of a composition or method, it may include substances or steps that do not substantially affect its basic properties in addition to the substances or steps described.

### ADVANTAGEOUS EFFECT

The composition of the present invention comprising a NOX1 inhibitor as an active ingredient exhibits an effect of alleviating tissue damage caused by ischemia-reperfusion through inhibition of reactive oxygen species (ROS)-mediated ERK signal transduction in an organ damage environment caused by ischemia-reperfusion, so it can be very usefully used for the prevention or development of therapeutic agents for organ damage caused by ischemia-reperfusion.

### BRIEF DESCRIPTION OF THE DRAWINGS

FIG. 1a to FIG. 1c is the result of confirming kidney damage through tissue staining and kidney function test of mice treated with ML171 in an animal experiment induced by oxidative stress.
FIG. 2a to FIG. 2e are the results of confirming the mRNA expression of NOX1 and 4 in the mouse, the expression of NOX4 in the tissue, and the generation of ROS by oxidative stress induction.
FIG. 3A to FIG. 3C are results of confirming apoptosis in a mouse model.
FIG. 4A to FIG. 4D are results of confirming renal fibrosis due to oxidative stress.
FIG. 5A and FIG. 5B are results of confirming the MAPK pathway gene by western blot to confirm the mechanism of renal cell death and fibrosis caused by oxidative stress.
FIG. 6A to FIG. 6F are results of confirming the ROS generation and NOX expression patterns of kidney cells by oxidative stress using MDCK cells.
In FIG. 7a to FIG. 7c, it was confirmed that ML171 blocks apoptosis of kidney cells by oxidative stress.
FIG. 8A and FIG. 8B are results of finding out the mechanism by which ML171 effectively blocks oxidative stress induction by H₂O₂ in cells.

### MODE FOR CARRYING OUT INVENTION

Hereinafter, the present invention will be described in detail by way of Examples. However, the following examples are only for illustrating the present invention, and the present invention is not limited thereto.

### Experimental method

C57BL/6 mice were intraperitoneally injected with 60 mg/kg of ML171, a selective NOX inhibitor or excipient at a time, renal ischemia-reperfusion injury (IRI) was induced by clamping bilateral renal vessels for 30 min. MDCK cells were incubated with H₂O₂ (1.4 mM) for 1 h to induce oxidative stress and treated with ML171 (1 and 2.5 µM). Renal damage was estimated using renal function tests and histology. NOX expression, oxidative stress markers, apoptosis assays and MAPK pathways were also evaluated in renal tissue and MDCK cells.

### Experiment result

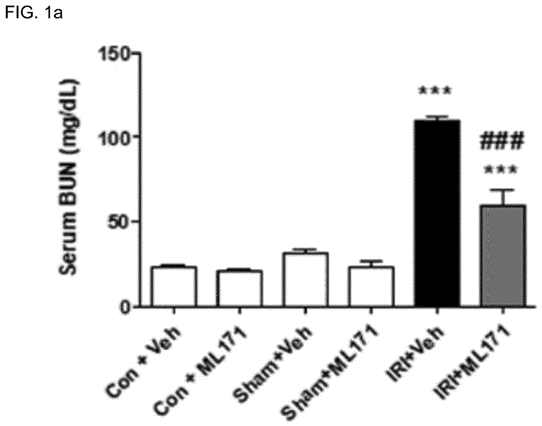

FIG. 1a to FIG. 1c show the results of confirming the kidney damage through the renal function test and tissue staining of mice treated with ML171 in an animal experiment induced by oxidative stress.

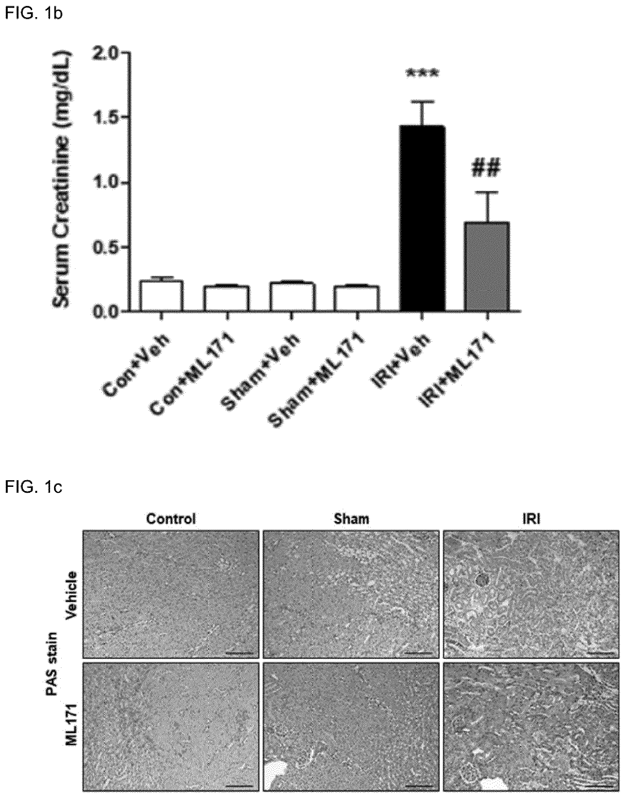

Referring to FIG. 1a and FIG. b, as a result of confirming the level of kidney damage in the blood, a significant decrease was confirmed in the mouse group treated with ML171, and referring to FIG. 1c, it was confirmed that ML171 reduced damage to renal tubular epithelial cells caused by oxidative stress even in histological analysis through PAS staining.

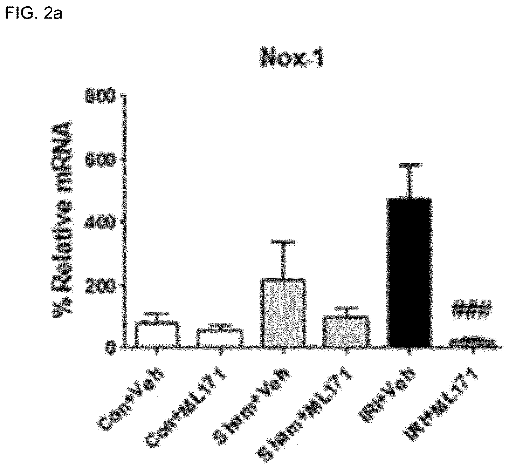

FIG. 2a to FIG. 2e are the results of confirming the mRNA expression of NOX1 and 4 in the mouse, the expression of NOX4 in the tissue, and the generation of ROS by oxidative stress induction.

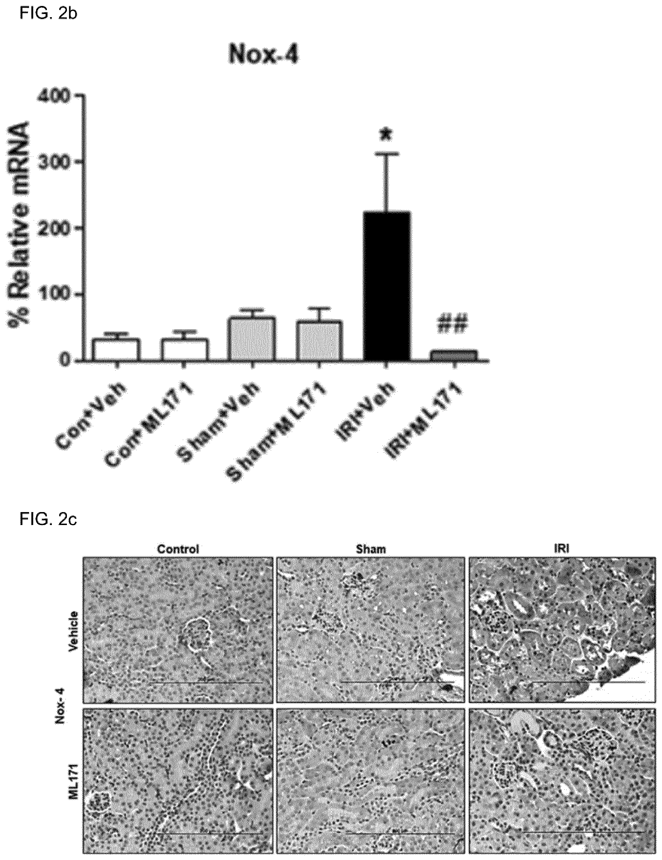

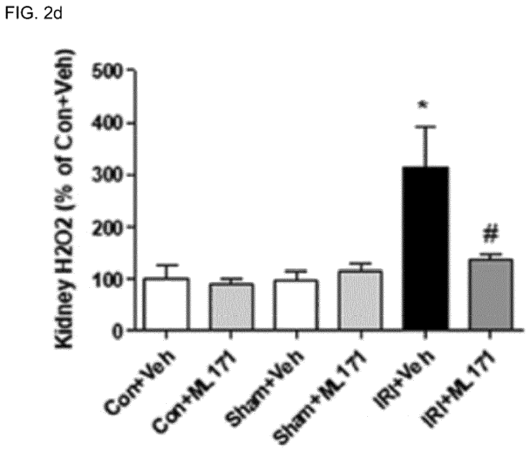

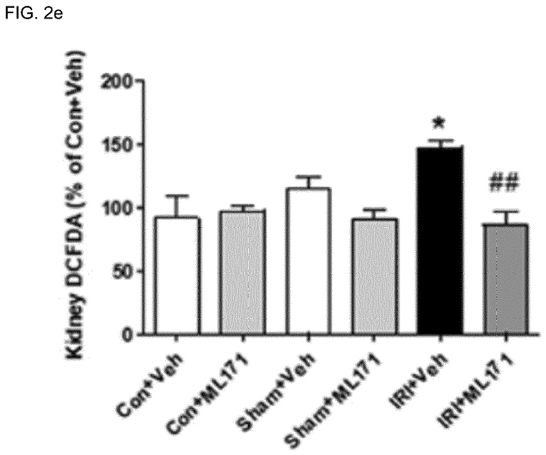

Referring to FIG. 2a and FIG. 2b, as a result of examining the mRNA expression level in mouse kidney, it was confirmed that NOX increased by oxidative stress was significantly reduced by ML171. Referring to FIG. 2c, it was confirmed that the expression level of NOX4 was increased in the IRI animal model even in the results of NOX4 immunostaining. Referring to FIG. 2d and FIG. 2e, as a result of measuring reactive oxygen species (ROS) in the kidney, it was confirmed that ML171 effectively blocked ROS, which significantly increased in the IRI group.

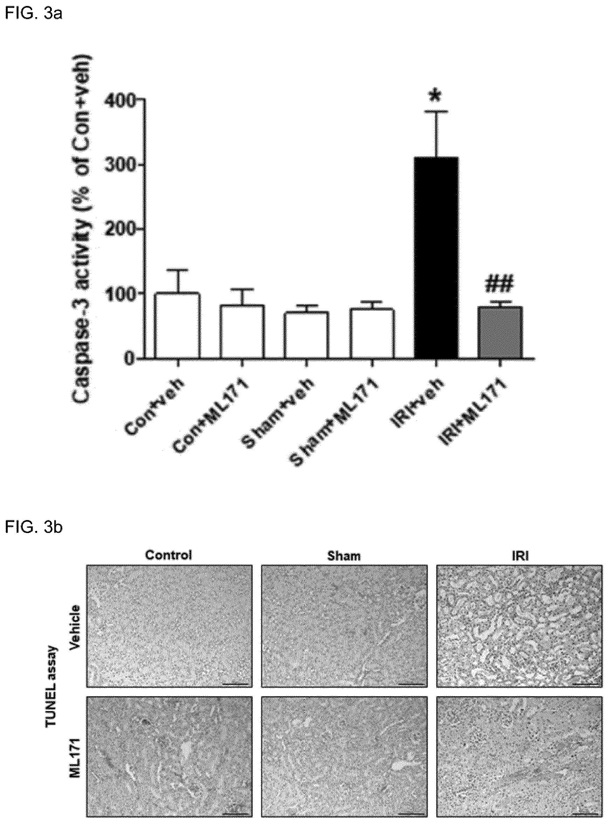

FIG. 3a to FIG. 3c are results confirming apoptosis in a mouse model.

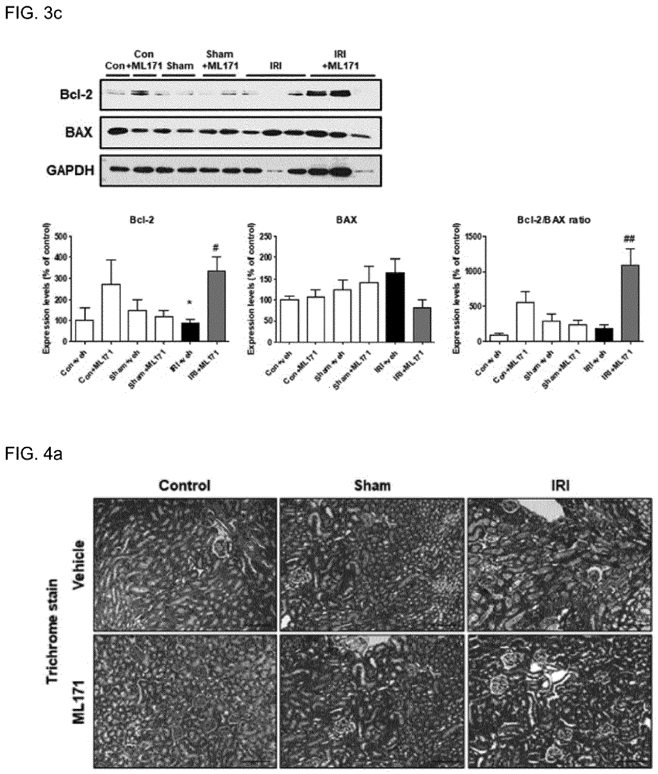

Referring to FIG. 3a, as a result of confirming Caspase-3 activity in the kidney, a significant apoptosis alleviation effect by ML171 was confirmed, and referring to FIG. 3b, it was confirmed that ML171 alleviated apoptosis caused by oxidative stress in the results of TUNEL assay histological staining. Referring to FIG. 3c, as a result of examining the protein expression levels in the kidneys of the apoptosis markers BCL-2 and BAX, it was confirmed that ML171 effectively inhibited apoptosis by IRI.

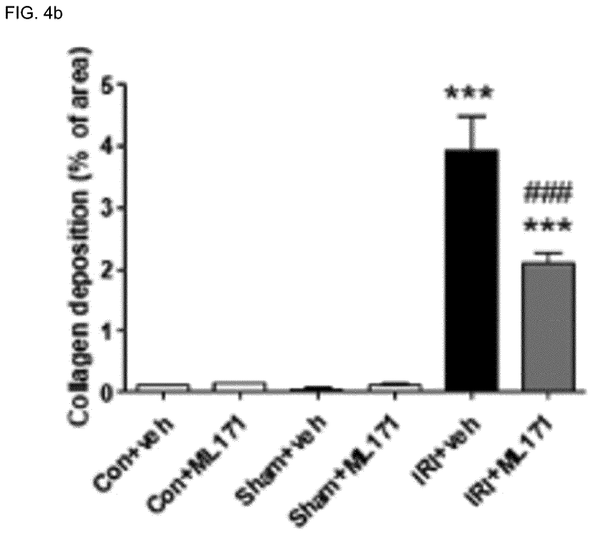

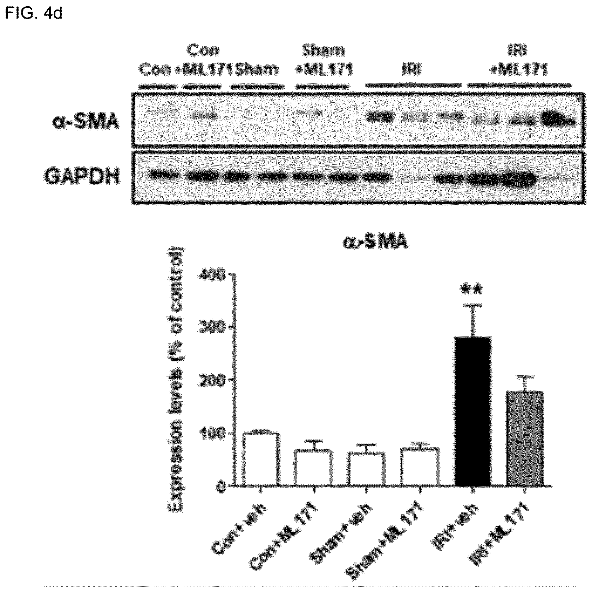

FIG. 4a to FIG. 4d are results of confirming renal fibrosis due to oxidative stress. Referring to FIG. 4a and FIG. 4b, as a result of confirming renal fibrosis through

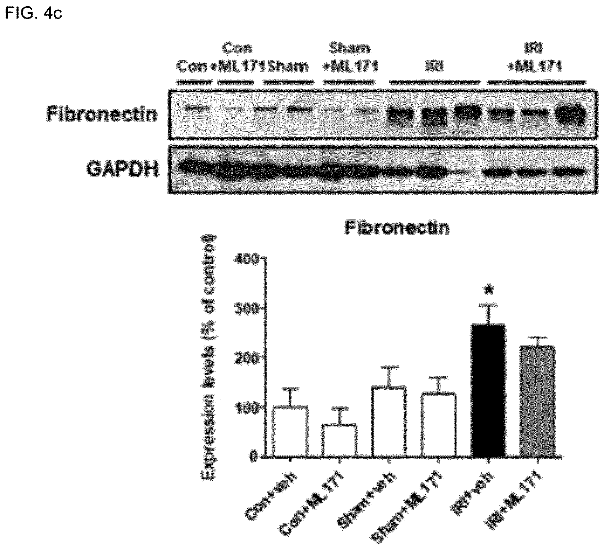

Trichrome stain, it was confirmed that fibrosis was significantly reduced by ML171, and referring to FIG. 4c and Referring to 4d, as a result of confirming the fibrosis markers Fibronectin and a-SMA markers, it was confirmed that the fibrosis progression was significantly increased by oxidative stress.

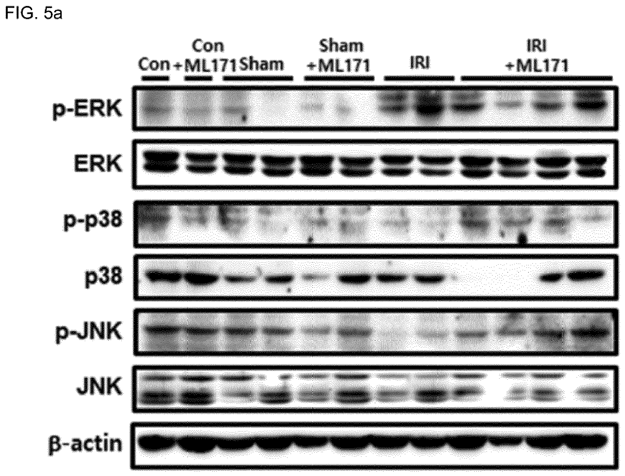

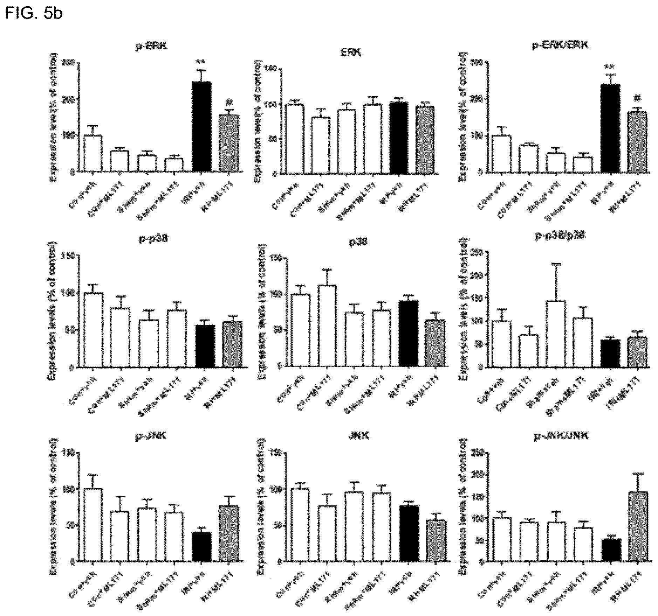

FIG. 5a and FIG. 5b are results of confirming the MAPK pathway gene by western blot to confirm the mechanism of renal cell death and fibrosis caused by oxidative stress.

As a result of confirming the gene of the MAPK pathway in FIG. 5a and FIG. 5b, it was confirmed that phosphorylation of ERK was significantly increased, and it was confirmed that ML171 effectively reduced it.

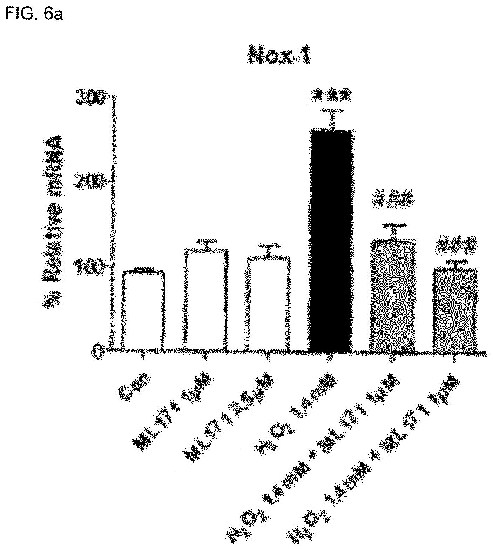

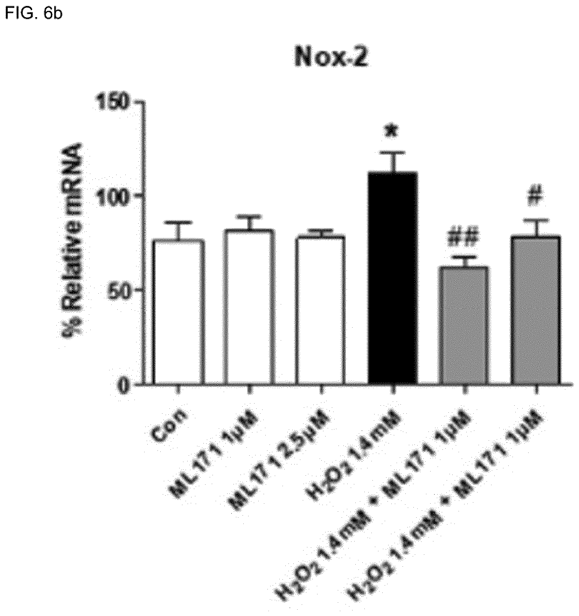

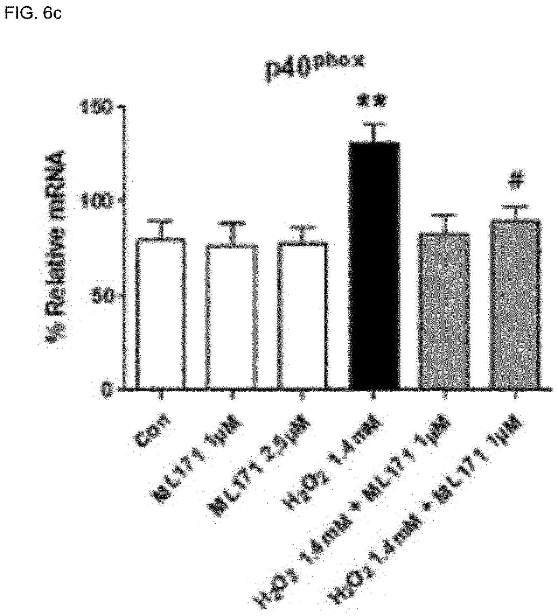

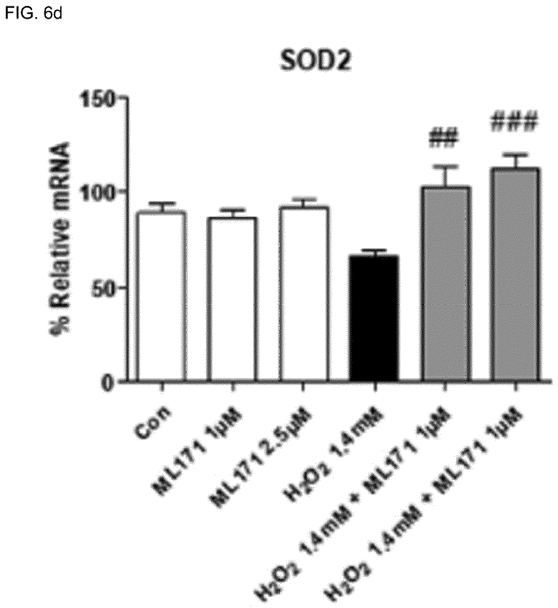

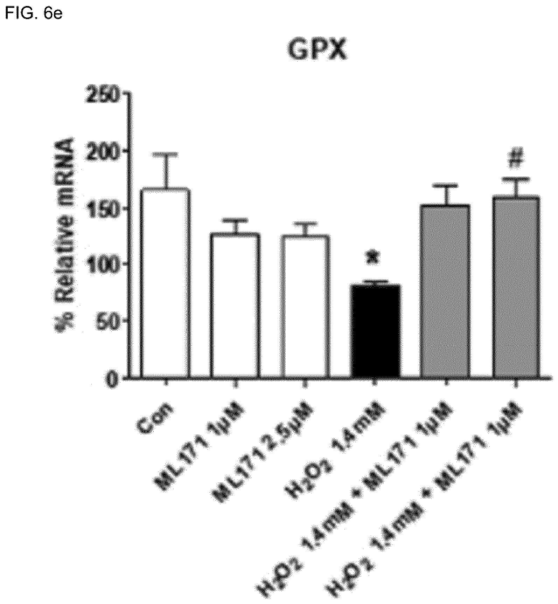

FIG. 6a to FIG. 6f are results of confirming the ROS generation and NOX expression patterns of kidney cells by oxidative stress using MDCK cells.

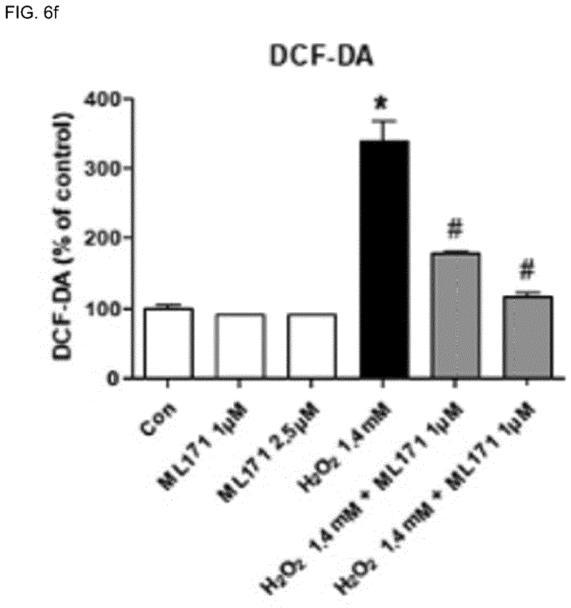

Referring to FIG. 6a to FIG. 6e, as a result of confirming the expression pattern of NOX-related genes after inducing oxidative stress through H₂O₂, it was confirmed that oxidative stress markers significantly increased by H₂O₂ were effectively decreased by ML171. Referring to FIG. 6f, it was confirmed that ML171 significantly reduced ROS, which was increased in cells by H₂O₂, even when ROS production was confirmed.

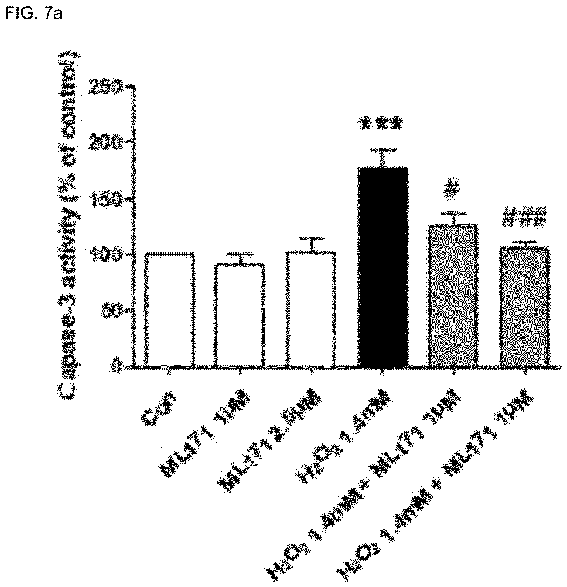

In FIG. 7a to FIG. 7c, it was confirmed that ML171 blocks apoptosis of kidney cells by oxidative stress.

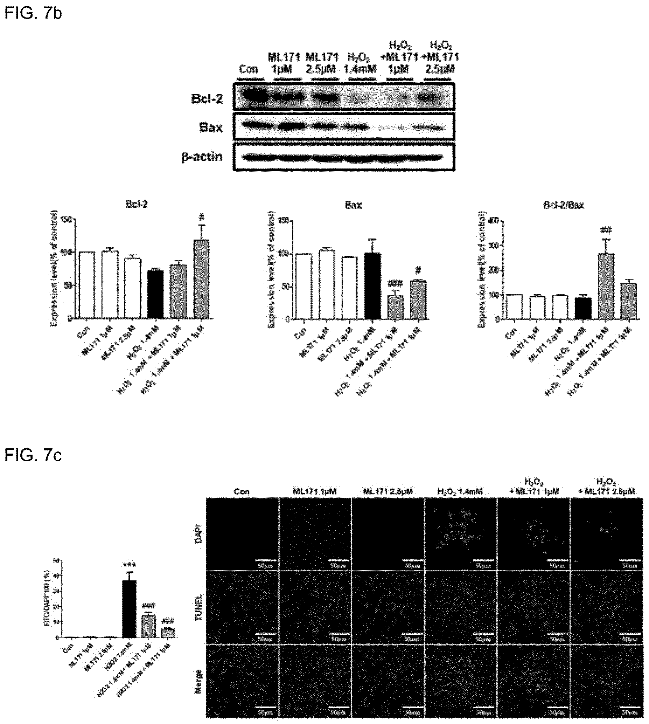

Referring to FIG 7a, it was confirmed that when oxidative stress by H₂O₂ was induced through Caspase-3 activity, apoptosis was significantly increased, and referring to FIG. 7b, the protein expression levels of BCL-2 and BAX, which are markers of apoptosis, were increased by H₂O₂, and it was confirmed that apoptosis was significantly inhibited by ML171. As a result of intracellular TUNEL assay staining of FIG. 7c, it was confirmed that the apoptosis pattern was increased by H₂O₂, and it was confirmed that ML171 effectively blocked it.

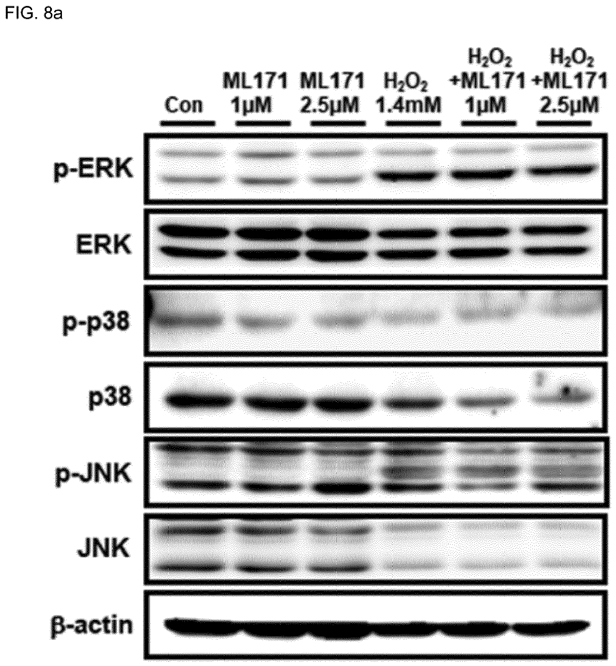

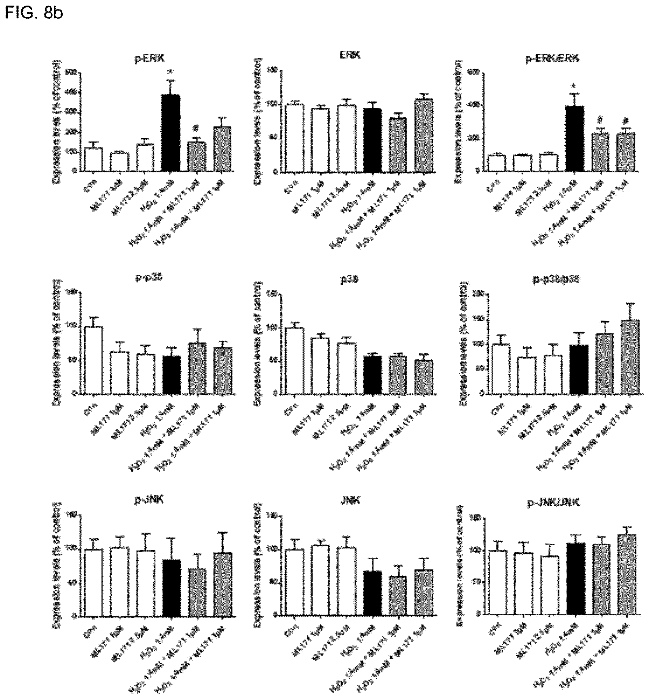

In FIG. 8a and In FIG. 8b are results of identifying the mechanism by which ML171 effectively blocks the induction of oxidative stress by H₂O₂ in cells.

As a result of confirming the MAPK pathway genes in MDCK cells, it was confirmed that oxidative stress-induced kidney damage was induced by ERK phosphorylation as in the in vivo experiment, and it was confirmed that ML171 significantly reduced and regulated this.

In conclusion, IRI worsened renal function and increased ROS production such as H₂O₂ and DCFDA in renal tissue, whereas treatment with ML171 significantly attenuated IRI-mediated damage. Intraperitoneal ML171 reversed a decrease in Bcl-2 and an increase in Caspase-3 activity. ML171 also reduced the expression of NOX1, NOX2 and p40 induced by H₂O₂ treatment in MDCK cells. H₂O₂ caused changes in oxidative stress-related enzymes in SOD and GXP production that were alleviated by ML171 treatment. ML171 caused a significant increase in Bcl-2 levels and a decrease in Caspase-3 activity. Among the MAPK pathways, ML171 was identified to affect ERK signaling by phosphorylation of ERK in kidney tissue and tubular cells.

### INDUSTRIAL APPLICABILITY

The composition of the present invention comprising a NOX1 inhibitor as an active ingredient exhibits an effect of alleviating tissue damage caused by ischemia-reperfusion through inhibition of reactive oxygen species (ROS) - mediated ERK signal transduction in an organ damage environment caused by ischemia-reperfusion, and thus can be very usefully utilized for the prevention or development of therapeutic agents for organ damage due to ischemia-reperfusion.

## Claims

1. A pharmaceutical composition for preventing or treating ischemia-reperfusion injury, comprising an NADPH oxidase 1 (NOX1) inhibitor as an active ingredient.

2. The pharmaceutical composition according to claim 1, wherein the ischemia-reperfusion injury is kidney injury.

3. The pharmaceutical composition according to claim 1, wherein the NOX1 inhibitor is a compound of Formula 1 below or a pharmaceutically acceptable salt thereof.

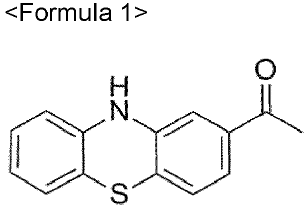

4. The pharmaceutical composition according to claim 1, wherein the NOX1 inhibitor is at least one selected from the group consisting of antisense nucleotides, siRNA, shRNA, and ribozyme for NOX1 gene.

5. The pharmaceutical composition according to claim 1, wherein the NOX1 inhibitor exhibits an ischemia-reperfusion injury preventing or therapeutic effect through inhibition of Reactive Oxygen Species (ROS) - mediated Extracellular-signal-Regulated Kkinase (ERK) signal transduction.

6. A food composition for preventing or improving ischemia-reperfusion injury, comprising an NADPH oxidase 1 (NOX1) inhibitor as an active ingredient.

7. The food composition according to claim 6, wherein the ischemia-reperfusion injury is kidney injury.

8. Use of a NADPH oxidase 1 (NOX1) inhibitor for the manufacture of a preparation for the treatment of ischemia-reperfusion injury.

9. The use according to claim 8, wherein the ischemia-reperfusion injury is a kidney injury.

10. The use according to claim 8, wherein the NOX1 inhibitor is a compound of Formula 1 below or a pharmaceutically acceptable salt thereof.

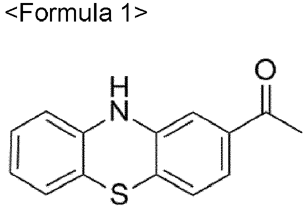

11. The use according to claim 8, wherein the NOX1 inhibitor is at least one selected from the group consisting of antisense nucleotides, siRNA, shRNA, and ribozyme for the NOX1 gene.

12. A method for treating ischemia-reperfusion injury comprising administering to a subject in need thereof an effective amount of a composition comprising an NADPH oxidase 1 (NOX1) inhibitor as an active ingredient.

13. The method according to claim 12, wherein the ischemia-reperfusion injury is a kidney injury.

14. The method according to claim 12, wherein the NOX1 inhibitor is a compound of Formula 1 below or a pharmaceutically acceptable salt thereof.

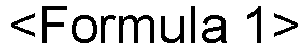

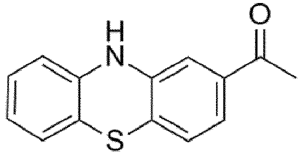

15. The method according to claim 12, wherein the NOX1 inhibitor is at least one selected from the group consisting of antisense nucleotides, siRNA, shRNA, and ribozyme for the NOX1 gene.
